Europäisches Patentamt
European Patent Office
Office européen des brevets

(19)

(11) Publication number: **0 339 498 A2**

(12) **EUROPEAN PATENT APPLICATION**

(21) Application number: **89107171.4**

(22) Date of filing: **20.04.89**

(51) Int. Cl.⁴: **C11D 1/37 , C11D 1/10 , A61K 7/08**

(30) Priority: **26.04.88 JP 103479/88**

(43) Date of publication of application: **02.11.89 Bulletin 89/44**

(84) Designated Contracting States: **DE FR GB IT**

(71) Applicant: **AJINOMOTO CO., INC.**
**5-8, Kyobashi 1-chome, Chuo-ku**
**Tokyo 104(JP)**

(72) Inventor: **Nakano, Hiroshi c/o Ajinomoto Co.,Inc.**
**No. 5-8, Kyobashi 1-chome Chuo-ku**
**Tokyo(JP)**
Inventor: **Ichiyanagi, Katsuyuki c/o Central Research**
**Ajinomoto Co.,Inc. No.1-1, Suzuki-ho**
**Kawasaki-ku Kawasaki-shi**
**Kanagawa-ken(JP)**

(74) Representative: **Strehl, Schübel-Hopf, Groening, Schulz**
**Maximilianstrasse 54 Postfach 22 14 55**
**D-8000 München 22(DE)**

(54) **Liquid detergent composition.**

(57) A liquid detergent composition which comprises:

(A) at least one N-acylated acidic amino acid represented by general formula (I) or a salt thereof;

$$M_1OOC-(CH_2)_m-\underset{\displaystyle R_1CONH}{\underset{|}{CH}}-COOM_2 \qquad (I)$$

wherein $R_1$ represents an alkyl group or an alkenyl group having 7 to 21 carbon atoms; $M_1$ and $M_2$ each represents hydrogen, an alkali metal or an alkanolammonium ion; and m represents an integer of 1 or 2,

and (B) at least one aliphatic sulfonic acid selected from compounds represented by general formula (II) or (III) or salts thereof;

$R_2-CH=CH(CH_2)_nSO_3M_3$ (II)

$$R_2-\underset{\displaystyle OH}{\underset{|}{CH}}-(CH_2)_nCH_2SO_3M_3 \qquad (III)$$

wherein $R_2$ represents an alkyl group having 7 to 15 carbon atoms; n represents 0 or an integer of 1 to 5; and $M_3$ represents hydrogen, an alkali metal or an alkanolammonium ion;

EP 0 339 498 A2

wherein the ratio (A)/(B) of component (A) to component (B) is 3/7 to 7/3 by weight and the total concentration of component (A) and component (B) in the composition is in a range of 4 to 30 wt%.

# LIQUID DETERGENT COMPOSITION

The present invention relates to liquid detergent compositions having excellent foaming properties, cleaning properties and safety and having good temperature stability. These compositions comprise N-acylated acidic amino acids or salts thereof and α-olefinsulfonic acids or salts thereof in a specific ratio.

In liquid detergents such as shampoo, facial soap, kitchen detergents, etc. anionic surfactants such as alkylbenzenesulfonates, higher alcohol sulfuric acid ester salts, polyoxyethylene alkyl ether sulfates, higher fatty acid salts, etc. which are excellent in foaming properties and cleaning properties have been hitherto formulated. However, these anionic surfactants are the cause of skin irritations and cause damages such as chapped hands, etc. In recent years, N-acylated acidic amino acid salts have been used in solid soap, cleansing foam and liquid detergents such as shampoo, kitchen detergents, etc. since the amino acid salts are highly safe to the human body, especially to the skin and show excellent touch in use.

In the case of utilizing N-acylated acidic amino acids in liquid detergents, however, alkali metal salts of N-acylated acidic amino acid have poor solubility so that these salts involve problems that insoluble matters are formed at low temperatures. On the other hand, the alkanolamine salts such as N-acylated acidic amino acid triethanolamine salt, etc. provide improved solubility but their foaming properties are insufficient in the presence of oil stains, as compared to the alkali metal salts; etc.

That is, an object of the present invention is to provide liquid detergent compositions which are excellent in foaming properties and cleaning properties and show further good temperature stability.

In view of the above described defects of detergents, the present inventors made extensive investigations to develop liquid detergents having improved foaming properties and cleaning properties, using N-acylated acidic amino acids or salts thereof. As a result, it has been found that by compounding N-acylated acidic amino acids or salts thereof and certain olefinsulfonic acids or hydroxyalkyl sulfonic acids or salts thereof in a specific ratio described below, liquid detergent compositions in which foaming properties and cleaning properties are improved and which are also excellent in temperature stability can be obtained.

The present invention is directed to a liquid detergent composition comprising:

(A) at least one N-acylated acidic amino acid represented by general formula (I) or a salt thereof;

$$M_1OOC-(CH_2)_m-\underset{R_1CONH}{\underset{|}{CH}}-COOM_2 \qquad (I)$$

wherein $R_1$ represents an alkyl group or an alkenyl group having 7 to 21 carbon atoms; $M_1$ and $M_2$ each represents hydrogen, an alkali metal or an alkanolammonium ion; and m represents an integer of 1 or 2,

and (B) at least one aliphatic sulfonic acid selected from compounds represented by general formula (II) or (III) or salts thereof;

$$R_2-CH=CH(CH_2)_nSO_3M_3 \qquad (II)$$

$$R_2-\underset{OH}{\underset{|}{CH}}-(CH_2)_nCH_2SO_3M_3 \qquad (III)$$

wherein $R_2$ represents an alkyl group having 7 to 15 carbon atoms; n represents 0 or an integer of 1 to 5; and $M_3$ represents hydrogen, an alkali metal or an alkanolammonium ion;

wherein the ratio (A)/(B) of component (A) to component (B) is 3/7 to 7/3 by weight and the total concentration of component (A) and component (B) in the composition is in a range of 4 to 30 wt%.

In the N-acylated acidic amino acids or salts thereof represented by general formula (I) described above which are used in the present invention, the acidic amino acids constituting the N-acylated acidic amino acids are exemplified by glutamic acid and aspartic acid. These amino acids may be any one of optically active and racemic compounds. As the acyl group (fatty acid residue), an aliphatic acyl group (fatty acid residue) having 8 to 22 carbon atoms can be used. Specific examples include capryloyl, caproyl, lauroyl, myristoyl, palmitoyl, stearoyl, behenoyl, oleoyl, cocoyl (coconut oil fatty acyl), hardened tallow fatty acyl, tallow fatty acyl, etc. These acyl groups may be used as admixture thereof.

Examples of the N-acylated acidic amino acids which can be n<ed in the present invention include N-capryloylglutamic acid, N-lauroylglutamic acid, N-myristoylglutamic acid, N-palmitoylglutamic acid, N-stearoylglutamic acid, N-behenoylglutamic acid, N-oleoylglutamic acid, N-cocoylglutamic acid, N-hardened

tallow fatty acylglutamic acid, N-caproylaspartic acid, N-lauroylaspartic acid, N-palmitoylaspartic acid, N-stearoylaspartic acid, N-behenoylaspartic acid, N-tallow fatty acylaspartic acid, etc. and mixtures thereof.

As the salts of N-acylated acidic amino acid used in the present invention, mention may be made of alkali metal salts such as sodium, potassium, lithium, etc. and triethanolamine, etc. salts. These salts may be used singly or as admixture thereof.

The olefinsulfonic acids (or salts thereof) represented by general formula (II) described above which are used in the present invention can be prepared by directly sulfonating olefins, such as α-olefins, having 10 to 22 carbon atoms with $SO_3$ gas diluted with an inert gas. The hydroxy alkyl sulfonic acids of formula III may also be produced in a conventional manner, e.g. by sulfurizing unsaturated alcohols with sulfuric acid or derivatives thereof. Examples of the α-olefinsulfonic acids, olefin sulfonic acids and hydroxyalkyl sulfonic acids of the formulae II and III include decenesulfonic acid, dodecenesulfonic acid, tetradecenesulfonic acid, hexadecenesulfonic acid, octadecenesulfonic acid, eicocenesulfonic acid, hydroxydecanesulfonic acid, hydroxydodecanesulfonic acid, hydroxytetradecanesulfonic acid, hydroxyhexadecanesulfonic acid, hydroxydoctaecanesulfonic acid, hydroxyeicosanesulfonic acid, etc. As the salts, mention may be made of lithium salts, sodium salts, potassium salts, triethanolamine salts, etc. In the present invention, these compounds or salts thereof may be used singly or as admixture thereof.

In the liquid detergent composition of the present invention, the ratio (A)/(B) of (A) the N-acylated acidic amino acids or salts thereof and (B) the compounds II or III or salts thereof is 3/7 to 7/3 by weight and the total concentration of component (A) and component (B) in the composition is in a range of 4 to 30 wt%, preferably 10 to 25 wt%. When the formulation ratio is outside such range, neither foaming properties nor cleaning properties are improved; alternatively, insoluble matters such as crystals, etc., particles produced by solidification, gellation, etc. are liable to occur so that temperature stability of liquid detergent compositions is not improved.

The liquid detergent composition of the present invention may suitably contain, in addition to the essential components described above, conventional raw materials for detergents such as various wetting agents, conditioners, builders, thickeners, flavors, coloring matters, antiseptics, etc., for example, glycerine, propylene glycol, 1,3-butylene glycol, polyethylene glycol, sodium d1-pyrrolidonecarboxylate, sodium lactate, urea, amino acids, protein, cationated polymers, lanoline or derivatives thereof, sodium citrate, sodium sulfate, hydroxyethyl cellulose, carboxyvinyl polymers, polyethylene glycol fatty acid esters, etc.

Furthermore, for purposes of controlling the touch or texture upon or after cleaning, other anionic surfactants, cationic surfactants, amphoteric surfactants or nonionic surfactants may also be appropriately incorporated within such a range that can achieve the objects of the present invention. Examples of these surfactants include long chain fatty acid salts, alkyl sulfates, polyoxyethylene alkyl ether sulfates, polyoxyethylene alkyl sulfosuccinates, acyl-monoethanolamide polyoxyethylenesulfosuccinic monoesters, monoalkylphosphates, polyoxyethylene alkyl ether phosphates, N-acylated neutral amino acids and salts thereof, quaternary ammonium salts, N-long chain acylated basic amino acid alkyl esters, 2-alkyl-carboxymethyl-N-hydroxyethyl imidazolinium betaines, higher fatty amide propyldimethylaminoacetic betaines, polyoxyethylene sorbitan fatty acid partial esters, polyoxyethylene glycerine fatty acid partial esters, polyoxyethylene mono-fatty acid esters, polyoxyethylene-hardened castor oil, sucrose fatty acid esters, N-acylglutamic acid polyoxyethylene alkyl ether diesters, monopyroglutamic mono-fatty acid polyoxyethylene-hardened castor oil, monopyroglutamic mono-fatty acid polyoxyethylene glycerines, fatty acid alkylolamides, alkylamine oxides, etc.

The liquid detergent composition of the present invention can be applied to produce hair shampoos, body shampoos, liquid facial detergents, kitchen detergents, liquid soap, etc. and many other liquid detergents.

Hereafter, the present invention is described in more detail by referring to the examples but is not deemed to be limited thereto. The effects are determined by the following

Skin irritation

Using 12 panel members, the procedure of dipping the hand in 2% aqueous solution (35°C) of the prepared liquid detergent compositon for a minute and then drying was repeated 20 times. Degree of chappiness was visually observed 24 hours after, based on the following criteria.

◎ : extremely weakly chapped (0 to 1 member gets chappy)
o : somewhat weakly chapped (2 to 3 members get chappy)
Δ : somewhat strongly chapped (4 to 6 members get chappy)
x : extremely strongly chapped (more than 7 members get chappy)

Foaming properties

In a 100 ml cylinder, 20 ml (35° C) of 5% aqueous solution of the prepared liquid detergent composition was taken and 0.2 g of artificial stain (mixture of 80 wt% of soybean oil and 20 wt% of squalene) was added to the solution. The cylinder was shaken 40 times for 20 seconds and the volume (ml) of foam was measured 1 minute after.

Cleaning properties

Using 5% aqueous solution of the prepared liquid detergent composition, cotton cloth (JIS L-0803) stained with artificial sebum was cleaned in Terg-O-Tometer according to JIS K-3371. A detergent efficiency D (%) was determined from reflectance of the cloth before and after the cleaning, by the following equation.

$D (\%) = [(C-B)/(A-B] \times 100$

A : reflectance of raw fabric (cotton cloth)

B : reflectance of stained cloth

C : reflectance of stained cloth after cleaning

The criteria for evaluation are defined as follows.

o : good cleaning property [$D \geqq 80$]

Δ : ordinary cleaning property [$80 \geqq D \geqq 60$]

X : poor cleaning property [$60 > D$]

Temperature stability

The prepared liquid detergent compositions were allowed to stand for 2 weeks at 0° C and 5° C, respectively. Change in color hue and odor, and change in solution such as occurrence of insoluble matters, solidification, etc. were observed. o : Change in color hue and odor or change in the solution was hardly noted.

Δ : Change in color hue and odor or change in the solution was somewhat noted.

x : Change in color hue and odor or change in the solution was considerably noted.

In each table, the composition is expressed by wt%.

Example 1

Liquid detergent compositions having various compositions shown in Tables 1, 2 and 3 were prepared to evaluate the efficiency.

Table 1

| Example 1: Run No. | | | 1 | 2 | 3 | | | | 4 | 5 | 6 | |
|---|---|---|---|---|---|---|---|---|---|---|---|---|
| Comparative Example 1: Run No. | 1 | 2 | | | | 3 | 4 | 5 | | | | 6 |
| Sodium N-lauroylglutamate (1.0 mol) | 10.0 | 8.0 | 7.0 | 5.0 | 3.0 | 2.0 | | 1.0 | 2.0 | 10.0 | 15.0 | 17.0 |
| Sodium tetradecenesulfonate/sodium hydroxytetradecanesulfonate (50:50 by weight) | | 2.0 | 3.0 | 5.0 | 7.0 | 8.0 | 10.0 | 1.0 | 2.0 | 10.0 | 15.0 | 17.0 |
| Purified water | 90.0 | 90.0 | 90.0 | 90.0 | 90.0 | 90.0 | 90.0 | 98.0 | 96.0 | 80.0 | 70.0 | 66.0 |
| Skin irritation | ◎ | ◎ | ◎ | ◎ | ◎ | o | Δ | ◎ | ◎ | ◎ | ◎ | ◎ |
| Foaming properties | 50 | 60 | 70 | 80 | 80 | 80 | 80 | 40 | 65 | 80 | 80 | 80 |
| Cleaning properties | Δ | Δ | o | o | o | o | o | x | o | o | o | o |
| Temperature stability | x | Δ | o | o | o | Δ | x | o | o | o | o | Δ |

Table 2

| Example 1: Run No. | 7 | 8 | 9 | 10 | 11 | 12 | 13 | 14 |
|---|---|---|---|---|---|---|---|---|
| Potassium N-myristoylglutamate (1.2 mol) | 5.0 | 10.0 | 5.0 | | | | | |
| N-Cocoylglutamate triethanolamine (1.1 mol) | | | | 10.0 | 10.0 | 5.0 | | |
| Lithium N-stearoylaspartate (1.5 mol) | | | | | | 5.0 | 5.0 | 10.0 |
| N-Lauroylglutamic acid | | | | | | | | 5.0 |
| Potassium dodecenesulfonate | 5.0 | 5.0 | 10.0 | | 5.0 | | | |
| Lithium octadecenesulfonate | | | | 10.0 | | | 5.0 | 10.0 |
| Hydroxyoctadecanesulfonate triethanolamine | | | | | 5.0 | | 5.0 | |
| Hydroxyhexadecanesulfonate | | | | | | 5.0 | | |
| Purified water | 90.0 | 85.0 | 85.0 | 80.0 | 80.0 | 85.0 | 85.0 | 75.0 |
| Skin irritation | ◎ | ◎ | ◎ | ◎ | ◎ | ◎ | ◎ | ◎ |
| Foaming properties | 75 | 80 | 80 | 80 | 80 | 80 | 80 | 80 |
| Cleaning properties | o | o | o | o | o | o | o | o |
| Temperature stability | o | o | o | o | o | o | o | o |

Table 3

| Example 1: Run No. | 15 | | | | | 16 | 17 |
|---|---|---|---|---|---|---|---|
| Comparative Example 1: Run No. | | 7 | 8 | 9 | 10 | | |
| Monosodium N-lauroylglutamate | 10.0 | 10.0 | 10.0 | | | 5.0 | 5.0 |
| Sodium dodecenesulfonate | 10.0 | | | 10.0 | 10.0 | 5.0 | 5.0 |
| Coconut oil fatty diethanolamide | | 10.0 | | 10.0 | | 10.0 | |
| Sodium polyoxyethylenelauryl ether sulfate | | | 10.0 | | 10.0 | | 10.0 |
| Purified water | 80.0 | 80.0 | 80.0 | 80.0 | 80.0 | 80.0 | 80.0 |
| Skin irritation | ◎ | ◎ | ◎ | Δ | x | ◎ | ◎ |
| Foaming properties | 80 | 60 | 60 | 80 | 80 | 80 | 80 |
| Cleaning properties | o | Δ | Δ | o | o | o | o |
| Temperature stability | o | x | x | Δ | o | o | o |

Results:

As is clear from the results shown in Tables 1, 2 and 3, the liquid detergent compositions of the prsent invention are excellent in safety, foaming properties, cleaning properties and temperature stability.

Example 2

The liquid soap compositions shown in Table 4 were prepared according to the present invention and tested by the methods described above.

Table 4.

| Liquid Soap Composition | |
|---|---|
| Component | Amount Formulated |
| N-Myristoylaspartate triethanolamine (1.3 mol) | 15.0 |
| Lithium tetradecenesulfonate | 10.0 |
| Glycerine | 3.0 |
| Benzalkonium chloride | 0.2 |
| Purified Water | balance |

Results:

The liquid soap compositions shown in Table 4 were excellent in safety, foaming properties, cleaning properties and temperature stability.

Example 3

The liquid facial soap compositions shown in Table 5 were prepared according to the prsent invention and tested by the methods described above.

Table 5.

| Liquid Facial Soap Composition | |
|---|---|
| Component | Amount Formulated |
| Monosodium N-cocoylglutamate | 10.0 |
| Sodium tetradecenesulfonate | 4.0 |
| Sodium hydroxytetradecanesulfonate | 6.0 |
| Glycerine monostearate | 1.0 |
| Propylene glycol | 5.0 |
| Glycerine | 3.0 |
| Methylparaben | 0.2 |
| Flavor | 0.2 |
| Purified Water | balance |

Results:

The liquid facial soap compositions shown in Table 5 were excellent in safety, foaming properties, cleaning properties and temperature stability.

Example 4

The hair shampoo compositions shown in Table 6 were prepared according to the present invention and tested by the methods described above.

Table 6.

| Hair Shampoo Composition | |
|---|---|
| Component | Amount Formulated |
| N-Cocoylglutamate monotriethanolamine | 5.0 |
| Monosodium N-lauroylglutamate | 4.0 |
| Sodium hydroxydodecanesulfonate | 6.0 |
| Cocoamide propyl betaine | 4.0 |
| Lauric acid diethanolamide | 5.0 |
| Sodium dl-pyrrolidonecarboxylate | 1.0 |
| Cationized cellulose | 0.2 |
| Methylparaben | 0.2 |
| Flavor | 0.2 |
| Purified Water | balance |

Results:

The liquid hair shampoo compositions shown in Table 6 were excellent in safety, foaming properties, cleaning properties and temperature stability.

Example 5

The body shampoo compositions shown in Table 7 were prepared according to the present invention and tested by the methods described above.

Table 7.

| Body Shampoo Composition | |
|---|---|
| Component | Amount Formulated |
| Monopotassium N-lauroylglutamate | 4.0 |
| N-Myristoylglutamic acid | 2.0 |
| Potassium tetradecanesulfonate | 8.0 |
| Disodium polyoxyethylenelaurylsulfosuccinate | 15.0 |
| 1,3-Butylene glycol | 5.0 |
| L-Arginine | 1.2 |
| Antiseptic | 0.2 |
| Flavor | 0.3 |
| Purified Water | balance |

Results:

The liquid body shampoo compositions shown in Table 7 were excellent in safety, foaming properties,

cleaning properties and temperature stability.

Example 6

The kitchen detergent compositions shown in Table 8 were prepared according to the present invention and tested by the methods described above.

Table 8.

| Kitchen Detergent Composition | |
|---|---|
| Component | Amount Formulated |
| Monolithium N-palmitoylglutamate | 6.0 |
| Lithium hydroxypentadecanesulfonate | 4.0 |
| Coconut oil fatty acid diethanolamide | 5.0 |
| Sodium polyoxyethylenelauryl ether sulfate | 8.0 |
| Laurylamine oxide | 10.0 |
| Glycerine | 5.0 |
| Urea | 1.0 |
| Antiseptic | 0.3 |
| Flavor | 0.2 |
| Purified Water | balance |

Results:

The kitchen detergent compositions shown in Table 8 were excellent in safety, foaming properties, cleaning properties and temperature stability.

[Effects of the Invention]

As described above, it is clear that the present invention provides liquid detergent compositions having excellent safety, foaming properties, cleaning properties and temperature stability.

## Claims

A liquid detergent composition comprising:

(A) at least one N-acylated acidic amino acid represented by general formula (I) or a salt thereof;

$$M_1OOC-(CH_2)_m-\underset{\displaystyle R_1CONH}{\underset{|}{CH}}-COOM_2 \qquad (I)$$

wherein $R_1$ represents an alkyl group or an alkenyl group having 7 to 21 carbon atoms; $M_1$ and $M_2$ each represents hydrogen, an alkali metal or an alkanolammonium ion; and m represents an integer of 1 or 2, and (B) at least one aliphatic sulfonic acid selected from compounds represented by general formula (II) or (III) or salts thereof;

$R_2-CH=CH(CH_2)_nSO_3M_3$ (II)

$R_2-\underset{\displaystyle OH}{\underset{|}{CH}}-(CH_2)_nCH_2SO_3M_3$ (III)

wherein $R_2$ represents an alkyl group having 7 to 15 carbon atoms; n represents 0 or an integer of 1 to 5; and $M_3$ represents hydrogen, an alkali metal or an alkanolammonium ion; wherein the ratio (A)/(B) of component (A) to component (B) is 3/7 to 7/3 by weight and the total concentration of component (A) and component (B) in the composition is in a range of 4 to 30 wt%.